(19) Europäisches Patentamt
European Patent Office
Office européen des brevets

(11) Publication number: 0 207 336
B1

(12) EUROPEAN PATENT SPECIFICATION

(45) Date of publication of patent specification: 02.01.91

(51) Int. Cl.$^5$: C 07 D 519/04, A 61 K 31/475

(21) Application number: 86107967.1

(22) Date of filing: 12.06.86

(54) **Nitro derivatives of vinblastine-type bis-indoles, a process for preparing same and pharmaceutical compositions containing them.**

(30) Priority: 12.06.85 HU 230285

(43) Date of publication of application:
07.01.87 Bulletin 87/02

(45) Publication of the grant of the patent:
02.01.91 Bulletin 91/01

(84) Designated Contracting States:
BE CH DE FR GB IT LI NL

(56) References cited:
DD-A- 236 094
DE-A-2 630 392
DE-A-2 815 822
FR-A-2 342 980

(73) Proprietor: RICHTER GEDEON VEGYESZETI GYAR R.T.
Gyömröi ut 19-21
H-1475 Budapest X (HU)

(72) Inventor: Szántay, Csaba, Dr.
Zsombolyai ut 8
H-1113 Budapest (HU)
Inventor: Szabó, Lajos, Dr.
Visegrádi u. 3/b
H-1132 Budapest (HU)
Inventor: Honty, Katalin, Dr.
Gébics u. 4/a
H-1124 Budapest (HU)
Inventor: Keve, Tibor, Dr.
Hunyadlejtö 28
H-1124 Budapest (HU)
Inventor: Acs, Tibor
Szilárd u. 92
H-1174 Budapest (HU)
Inventor: Eckhardt, Sándor, Dr.
Julia u. 1
H-1026 Budapest (HU)
Inventor: Sugár, János, Dr.
Matróz u. 6
H-1051 Budapest (HU)

EP 0 207 336 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Somfai, Szuzsa, Dr.**
**Október 6. u. 16**
**H-1051 Budapest (HU)**
Inventor: **Iván, Eva, Dr.**
**Hajnóczi u. 6**
**H-1122 Budapest (HU)**
Inventor: **Kneffel, Zsuzsa, Dr.**
**Ajtósi Dürer sor. 7**
**H-1146 Budapest (HU)**

(74) Representative: **Beszédes, Stephan G., Dr.**
**Patentanwalt**
**Münchener Strasse 80a Postfach 1168**
**D-8060 Dachau (DE)**

**Description**

The invention is concerned with novel nitro derivatives of vinblastine-type bis-indoles, a process for preparing same and pharmaceutical compositions, particularly having a cytostatic effect.

It has been known from the literature that hitherto relatively few compounds have been described in which the aromatic nucleus of the bis-indole alkaloid derivative was substituted. Among these compounds, the 15',20'-(anhydro)-7'-(chloro)-vinblastine (Chem. Abstr. *94* [1981], 30987k, the 10'-(hydroxy)-leurosine (J. Nat. Prod. *44* [1981], 478), as well as the 10'-(iodo)-vinblastine (United States patent specification 4,430,269) are remarkable.

No literature data, however, are available on nitro-bis-indole derivatives substituted on their aromatic nucleus.

The problem underlying to the invention is to create new derivatives of vinblastine-type bis-indoles, a process for preparing same and pharmaceutical compositions, particularly having a cytostatic effect, containing them.

Surprisingly the above has been attained by the invention.

Now it has been found in the course of our investigations that various nitro derivatives, particularly mononitro derivatives, of vinblastine-type bis-indoles have an excellent pharmaceutical activity, particularly against tumours, and these are formed in excellent yields when the known bis-indole alkaloids are nitrated, particularly by using fuming nitric acid in a solvent mixture consisting of glacial acetic acid and chloroform.

Hence a subject-matter of the invention are nitro derivatives of vinblastine-type bis-indoles of the general formula

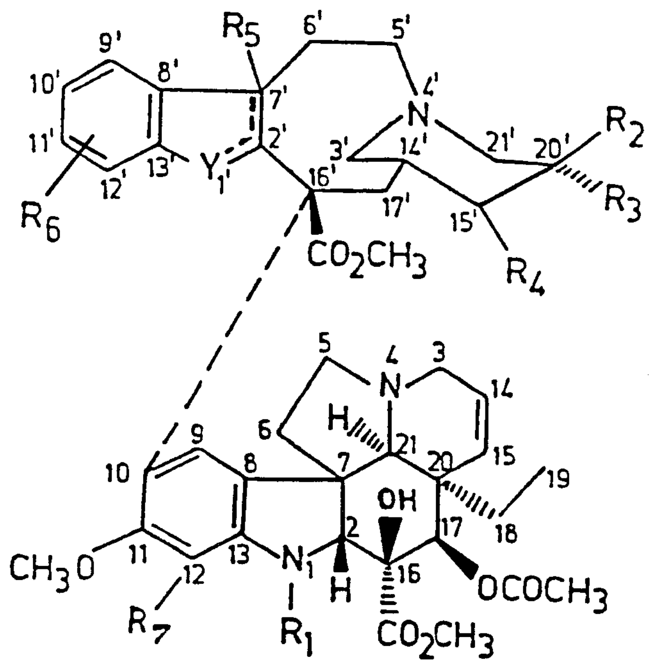

I,

wherein

$R_1$ stands for a methyl or a formyl group,

$R_2$ stands for a hydroxy or ethyl group of β-position,

$R_3$ means an ethyl group of α-position,

$R_4$ represents a hydrogen atom or

$R_2$ and $R_4$ together represent an oxygen bridge or a double bond,

$R_5$, $R_6$ and $R_7$ stand for a nitro group or a hydrogen atom with the proviso that simultaneously only at most two of them may stand for [a] hydrogen atom(s), and

Y stands for —N= when $R_5$ stands for a nitro group thus the dotted line between Y and the carbon atom 2' representing a valence bond and the dotted line between the carbon atoms 2' and 7' having no meaning and

Y stands for

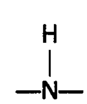

when $R_5$ stands for a hydrogen atom thus the dotted line between Y and the carbon atom 2' having no

meaning and the dotted line between the carbon atoms 2′ and 7′ representing a valence bond, as well as their acid addition salts.

The French patent publication 2 342 980 is concerned with a process for preparing 9-aminoapovincamine and 11-aminoapovincamine by nitration of apovincamine and subsequent reduction of the 9-nitroapovincamine and 11-nitroapovincamine, respectively, obtained thereby. These compounds which in terms of their structure are differing considerably from the nitro-bis-indolo compounds of the vinblastine type of the present application are used as regulators of the cerebral circulation.

The German published patent application 26 30 392 describes dimeric anhydrovincaalkaloides and their preparation. These substances differ from the compounds of the present invention especially in terms of the substituents $R^2$ and $R^3$ and optionally $R^5$, $R^6$ and $R^7$ in the case of the presence of the latters, and by the absence of an ethyl group in the 20-position of compounds of the present invention, and additionally of a nitro group.

These compounds are intermediates for the preparation of oncogenetic active alkaloides.

The German published application 28 15 822 describes a process for preparing bis-indolo compounds of the leurosine type and their derivatives. These compounds differ from the compounds of the present invention by the absence of the nitro group. These known compounds are used as intermediates for the preparation of pharmaceuticals having antitumour activity.

Particularly preferred compounds according to the invention are 12-(nitro)-vinblastine, 11′-(nitro)-vincristine, 9′-(nitro)-vincristine, 7′-(nitro)-vincristine, N-(formyl)-11′-(nitro)-leurosine, N-(formyl)-7′-(nitro)-leurosine and 15′,20′-(anhydro)-11′-(nitro)-vincristine as well as the acid addition salts of these compounds.

A subject-matter according to the invention is also a process for preparing the compounds according to the invention which is characterized in that

a) a compound of the general formula

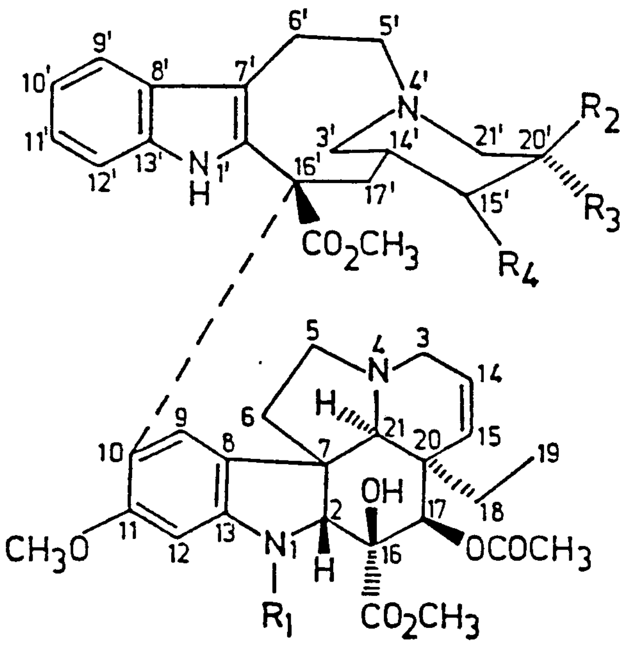

II,

wherein $R_1$, $R_2$, $R_3$ and $R_4$ are the same as defined above, is nitrated in an organic solvent, preferably in a chlorinated hydrocarbon, in the presence of an organic acid, preferably acetic acid, and optionally

b) a compound of the general formula I, wherein $R_1$, $R_3$, $R_5$, $R_6$, $R_7$ and Y are the same as defined above, $R_2$ means a hydroxy group and $R_4$ represents a hydrogen atom, is dehydrated and the thus obtained compound is oxidized, whereby the meaning of $R_2$ and $R_4$ together is changed to an oxygen bridge and, if desired, the thus obtained product is transformed to an acid addition salt thereof.

Preferably the reaction a) of the process according to the invention is carried out as follows. The starting material of the general formula II is dissolved in a solvent, particularly in a mixture of chloroform with glacial acetic acid. Fuming nitric acid is added to this solution at a temperature of about −20°C (−20 ± 2°C), then the reaction mixture is poured into ice-water. After alkalization and extraction, the crude product is purified by layer or column chromatography.

On carrying out the reaction b) of the process according to the invention a compound of the general formula I is chosen which contains a nitro group bound to the aromatic nucleus and can be transformed by a simple chemical operation, i.e. by dehydration and oxidation.

After accomplishing the above reactions, the product may be obtained from the reaction mixture by extraction and/or evaporation and, if desired, purified by using a chromatographical and/or crystallization method. The chromatography is carried out advantageously on a sheet or column prepared with silica gel.

According to a preferred embodiment of the invention for the preparation of 11'-(nitro)-vincristine, vincristine base is reacted with fuming nitric acid in the presence of chloroform and acetic acid and the formed product is separated.

The starting materials used in reaction a) of the process according to the invention are known compounds.

The starting materials used in reaction b) of the process according to the invention are new compounds which are prepared according to the reaction a) of the process according to the invention.

A subject-matter of the invention are also pharmaceutical compositions characterized by a content of 1 or more compound(s) according to the invention as [an] active principle(s), suitably in admixture with 1 or more usual pharmaceutical carrier(s) and/or additive(s).

The compounds of the general formula I show a cytostatic effect with less toxicity than that of the known vinblastine-type bis-indole alkaloid drugs which are commercially available.

For investigating the biological activity, the solutions were prepared by using physiological saline solution and, if desired, in the case of bases, by adding one drop of Tween-80®. The injectable solutions were intraperitoneally administered in a volume of 0.1 ml/10 g of body-weight.

The activity of the novel compounds of the invention was investigated on intraperitoneally transplantable tumours [P388 mouse leukaemia) by using the method described hereinafter.

The P388 leukaemia was maintained in DBA2 inbred mice and transplanted intraperitoneally by administering $10^6$ tumour cells/animal to groups consisting of 6 $BDF_1$ hybride mice each. In the 24th hour following the transplantation, the treatment with the compounds to be tested was started with daily intraperitoneal injections for 8 days. The body-weight, condition and tumour of the animals were daily controlled. The effect was measured on lengthening the life span in days and expressed as the percentage of the mean survival time of the treated group as related to that of the control group (T/C%).

It is obvious from the Table that the compounds of the invention advantageously influence the life span of the mice suffering from P388 leukaemia. It can be seen that the broadness of the action of 11'-(nitro)-vincristine is preferred. A single daily dose of 8 to 16 mg/kg of body weight and even a high single dose of 20 to 40 mg/kg of body weight was tolerated by the animals without any paralysis or mortality, respectively, as opposed to vincristine which is only effective when used in repeated doses.

TABLE

| Compound | Dose mg/kg i.p. | Mean survival time Treated days | Control days | T/C% |
|---|---|---|---|---|
| 12-(Nitro)-vinblastine | 8 × 4.0 | 11.4 | 9.0 | 127 |
| | 8 × 8.0 | 15.4 | 9.6 | 160 |
| | 8 × 16.0 | 15.6 | 9.6 | 163 |
| 7'-(Nitro)-vincristine | 8 × 2.0 | 11.0 | 9.6 | 115 |
| | 8 × 8.0 | 16.8 | 9.6 | 175 |
| | 1 × 20.0 | 16.0 | 9.6 | 167 |
| 11'-(Nitro)-vincristine | 8 × 0.4 | 12.0 | 9.8 | 122 |
| | 8 × 1.0 | 14.0 | 9.8 | 143 |
| | 8 × 2.0 | 16.8 | 9.8 | 171 |
| | 8 × 8.0 | 20.8 | 9.6 | 217 |
| | 8 × 12.0 | 19.0 | 9.2 | 207 |
| | 1 × 20.0 | 15.2 | 9.2 | 165 |
| | 1 × 40.0 | 18.0 | 9.2 | 196 |
| 11'-(Nitro)-vincristine sulphate | 8 × 1.0 | 14.4 | 10.0 | 144 |
| | 8 × 4.0 | 18.2 | 10.0 | 182 |
| | 8 × 8.0 | 16.0 | 9.2 | 174 |
| Vincristine (a known substance) [reference] | 8 × 0.05 | 11.1 | 9.0 | 123 |
| | 8 × 0.1 | 18.7 | 9.2 | 203 |
| | 8 × 0.2 | 16.8 | 9.2 | 183 |
| | 8 × 0.4 | 21.7 | 9.2 | 236 |

The invention is illustrated in detail by the aid of the following Examples.

Example 1

Preparation of 12-nitrovinblastine by the nitration of vinblastine

A solution containing 0.09 ml (0.136 g, 2.16 mmoles, 3.3 equivalents) of fuming nitric acid (d = 1.5) in 0.38 ml of glacial acetic acid is dropped to a solution containing 0.53 g (0.65 mmole) of vinblastine in a solvent mixture of 1.25 ml of glacial acetic acid and 1 ml of abs. chloroform within 10 minutes under constant stirring and maintaining the temperature at −20°C. Then the reaction mixture is poured into 10 ml of ice-water, alkalized to pH 9 by adding concentrated ammonium hydroxide solution and extracted 3 times with 5 ml of dichloromethane each. After drying over anhydrous magnesium sulphate, the organic solution is filtered and evaporated to give 0.50 g of an oily product which is purified by preparative thin layer chromatography (TLC) on a KG—$PF_{254+366}$ sheet by using a developing system containing dichloromethane and methanol in a volume ratio of 20:2. The $R_f$ value of vinblastine is lower than that of the nitro derivative which is in turn lower than that of the oxidized product. The elution is carried out with a mixture containing dichlormethane and methanol in a volume ratio of 20:4. The aimed compound is obtained in a yield of 2.75

mg (49%); m.p.: decomposition beginning from 200°C; $[\alpha]_{546}^{21} = -234°$; $[\alpha]_D^{21} = -212°$ (c = 1.00, chloroform).

IR (KBr, $cm^{-1}$): 3460 (NH, OH), 1735 (ester CO), 1520, 1375 ($NO_2$).

MS m/z (%): 869 (M + 14, with an intensity changing with time), 855 ($M^+$, 3, $C_{46}H_{57}N_5O_{11}$), 825 (5), 824 (8), 696 (1), 367 (10), 355 (8), 294 (5), 155 (50), 154 (67), 122 (52), 121 (75), 107 (60), 106 (100).

$^1$H—NMR ($CDCl_3$): δ = 8.02 (indole NH), 7.52—7.10 (4H, m, 9'-, 10'-, 11'-, 12'-H), 6.82 (1H, s 9-H), 5.87 (1H, dd, 14-H), 5.45 (1H, s, 17-H), 5.35 (1H, d, 15-H), 3.87 (1H, s, 2-H), 3.77, 3.72, 3.66 (9H, s, $CO_2CH_3$, $OCH_3$), 2.82 (2H, 21'-H), 2.63 (3H, s, $NCH_3$), 2.16 (3H, s, $OCOCH_3$), 0.88 (3H, t, 18'-$CH_2\mathit{CH_3}$), 0.76 ppm (3H, t, 18'-$CH_2\mathit{CH_3}$).

## Example 2

Preparation of 11'-nitrovincristine, 9'-nitrovincristine and 7'-nitrovincristine by the nitration of vincristine

A solution containing 0.15 ml (0.228 g, 3.6 mmoles, 3.5 equivalents) of fuming nitric acid (d = 1.5) in 0.65 ml of glacial acetic acid is dropped to a solution containing 0.85 g (1.03 mmoles) of vincristine in a mixture of 2.65 ml of abs. chloroform and 2.10 ml of glacial actic acid at −20°C, whereupon the stirring is continued at the same temperature for additional 3 hours. Then the mixture is poured into 60 ml of ice water, alkalized to pH 9 by adding concentrated ammonium hydroxide solution and extracted 3 times with 20 ml of dichloromethane each. After drying over anhydrous magnesium sulphate, filtering and evaporating, the thus-obtained product weighing 0.89 g is separated by using preparative TLC on a KG—$PF_{254+366}$ sheet by means of a developing system containing dichloromethane and methanol in a volume ratio of 20:2. The elution is carried out with a mixture of dichloromethane and methanol in a volume ratio of 20:4. The order of the $R_f$ values is as follows: 7'-nitrovincristine > 9'-nitrovincristine > 11'-nitrovincristine > vincristine. By using the above method, the 11'-, 9'- and 7'-nitro derivatives can be separated.

11'-Nitrovincristine is obtained in a yield of 0.54 g (60%); m.p.: 225°C (with decomposition); $[\alpha]_D^{21} = +92°$; $[\alpha]_{546}^{21} = +112°C$ (c = 1.00, chloroform).

IR (KBr, $cm^{-1}$): 3360 (NH, OH), 1725 (ester CO), 1660 (NCHO).

MS m/z (%): 883 (M+14, 0.4), 869 ($M^+$, 1.0, $C_{46}H_{55}N_5O_{12}$), 852 (0.6), 838 (2.1), 393 (1.3), 355 (1.0), 302 (5.5), 205 (3.8), 154 (40), 141 (20), 136 (12), 122 (16), 120 (6.9), 106 (14), 74 (79), 72 (92).

$^1$H—NMR ($CDCl_3$): δ = 8.7, 8.56 (1H, NCHO), 8.60 (1H, s, indole NH), 8.13 (1H, $J_m$ = 2 Hz, 12'-H), 7.97 (1H, $J_o$ = 9 Hz. $J_m$ = 2 Hz, 10'-H), 7.54 (1H, $J_o$ = 9 Hz, 9'-H), 6.76 (1H, s, 9-H), 5.92 (1H, dd, 14-H), 5.42 [(1H, d, 15-H), 5.22 (1H, s, 17-H), 4.62 (1H, s, 2-H), 3.86, 3.72, 3.68 (9H, s, $CO_2CH_3$, $OCH_3$), 2.04 (3H, s, $OCOCH_3$), 0.88 (3H, t, 18-$CH_2\mathit{CH_3}$), 0.76 (3H, t, 18'-$CH_2\mathit{CH_3}$).

From the middle zone, 9'-nitrovincristine is isolated in a yield of 50 mg (5%); m.p.: 220°C (with decomposition); $[\alpha]_{546}^{27} = +109.5°$ (c = 1.00, chloroform).

IR (KBr, $cm^{-1}$): 3480 (NH, OH), 1730 (ester CO), 1680 (NCHO).

MS m/z (%): 883 (M + 14, 0.05), 869 ($M^+$, 1.0, $C_{46}H_{55}N_5O_{12}$).

$^1$H—NMR ($CDCl_3$): δ = 8.62 (1H, s, indole NH), 8.52 (1H, br, NHCO), 7.45 (1h, $J_o$ = 8 Hz, $J_m$ = 1.5 Hz, 10'-H), 7.35 (1H, $J_o$ = Hz, $J_m$ = 1.5 Hz, 12'-H), 7.13 (1H, dd, $J_o$ = 8, 8.5 Hz, $J_m$ = 1.5 Hz, 11'-H), 5.90 (1H, dd, 14-H), 5.42 (1H, d, 15-H), 5.22 (1H, s, 17-H), 4.63 (1H, s, 2-H), 3.85, 3.73, 3.67 (9H, s, $CO_2CH_3$, $OCH_3$), 2.02 (3H, s, $OCOCH_3$), 0.84 (3H, t, 18-$CH_2\mathit{CH_3}$), 0.65 ppm (3H, t, 18'-$CH_2\mathit{CH_3}$).

Finally, 7'-nitrovincristine is obtained in a yield of 0.28 g (31%) from the zone characterized by the highest $R_f$ value, m.p.: 212°C (with decomposition) after recrystallization from methanol; $[\alpha]_D^{20} = -95°$; $[\alpha]_{546}^{20} = -112°$ (c = 1.00, chloroform).

IR (KBr, $cm^{-1}$): 3420 (OH), 1735 (ester CO), 1678 (NCHO), 1552 (C = N).

MS m/z (%): 869 ($M^+$, 1.0, $C_{46}H_{55}N_5O_{12}$), 838 (6.4), 393 (8.4), 391 (3.7), 355 (12), 205 (17), 182 (21), 154 (87), 141 (37), 136 (36), 122 (51), 121 (88), 120 (28).

$^1$H—NMR ($CDCl_3$): δ = 8.46 (1H, NCHO), 7.70 (1H, s, 12-H), 7.46—6.90 (5H, m, aromatic protons), 5.92 (1H, dd, 14-H), 5.42 (1H, d, 15-H), 5.22 (1H, s, 17-H), 4.70 (1H, s, 2-H), 3.75, 3.42 (9H, s, $CO_2CH_3$, $OCH_3$), 2.00 (3H, s, $OCOCH_3$), 0.88 (3H, t, 18-$CH_2\mathit{CH_3}$), 0.79 (3H, t, 18'-$CH_2CH_3$).

## Example 3

Preparation of N-formyl-11'-nitroleurosine and N-formyl-7-nitroleurosine by the nitration of N-formylleurosine

A solution containing 0.16 ml (0.24 g, 3.85 mmoles) of fuming nitric acid in 0.68 ml of glacial acetic acid is dropped to a solution containing 0.89 g (1.08 mmoles) of N-formylleurosine base in the solvent mixture of 2.19 ml of glacial acetic acid and 14 ml of abs. chloroform at −20°C, then the stirring is continued at the same temperature for additional 3 hours, whereupon the reaction mixture is poured into 10 ml of ice-water, alkalized to pH 9 by adding concentrated ammonium hydroxide solution and extracted 3 times with 30 ml of dichloromethane each. After drying over anhydrous magnesium sulphate, filtering and evaporating, the thus-obtained oily product weighing 0.68 g is separated by using preparative TLC on KG—$PF_{254+366}$ sheet by means of a developing system containing dichlormethane and methanol in a volume ratio of 20:2. The $R_f$ value of N-formyl-11'-nitroleurosine is higher than that of the starting N-formylleurosine. The elution is carried out with a mixture of dichloromethane and methanol in a volume ratio of 20:4.

N-Formyl-11'-nitroleurosine is obtained in a yield of 0.23 g (24.5%); m.p.: 215°C (with decomposition), after rubbing with ether; $[\alpha]_D^{21} = +105°$; $[\alpha]^{21}_{546} = +136°$ (c = 1.00, chloroform).

IR (KBr, $cm^{-1}$): 3400 (OH), 1730 (ester CO), 1660 (NCHO).

$^1$H-NMR ($CDCl_3$): δ = 8.72 (1H, s, indole-NH), 8.13 (1H, $J_m$ = 2 Hz, 12'-H), 7.96 (1H, $J_o$ = 9 Hz, $J_m$ = 2 Hz, 10'-H), 7.54 (1h, $J_o$ = 9 Hz, 9'-H), 7.2—6.9 (1H, 12-H), 6.79 (1H, s, 9-H), 5.96 (1H, dd, 14-H), 5.46 ([1h, d, 15-H), 5.22 (1H, s, 17-H), 4.65 (1H, s, 2-H), 3.88, 3.73, 3.68, (9H, s, $CO_2CH_3$, $OCH_3$), 2.06 (3H, s, $OCOCH_3$), 0.95 (3H, t, 18-$CH_2$*$CH_3$*), 0.74 ppm (3H, t, 18'-$CH_2$*$CH_3$*).

From the zone characterized by the higher $R_f$ value, N-formyl-7'-nitroleurosine can be obtained in a yield of 70 mg (7%); m.p.: 219°C (with decomposition) after rubbing with ether; $[\alpha]_D^{21} = -77°$; $[\alpha]_{546}^{21} = -83°$ (c = 1.00, chloroform).

$^1$H—NMR ($CDCl_3$): δ = 8.54 (1H, br, NCHO), 7.90 (1H, s, 12-H), 7.50—7.18 (5H, m, aromatic protons), 5.85 (1H, dd, 14-H), 5.27 (1H, d, 15-H), 5.12 (1H, s, 17-H), 4.68 (1H, s, 2-H), 3.76, 3.74, 3.68 (9H, s, $CO_2CH_3$, $OCH_3$), 1.94 (3H, s, $OCOCH_3$), 0.92 (3h, t, 18-$CH_2$*$CH_3$*), −0.12 ppm (3H, t, 18'-$CH_2$*$CH_3$*).

## Example 4

### Preparation of 15',20'-anhydro-11'-nitrovincristine

Method a)

A solution containing 0.4 ml of thionyl chloride in 1 ml of dimethyl formamide is added to the solution of 0.20 g (0.23 mmole) of 11'-nitrovincristine in 3 ml of dimethyl formamide under cooling with ice. The mixture is allowed to stand at 0°C for 20 minutes and then at room temperature for additional 2 hours. Thereupon, the mixture is poured into 30 ml of ice-water, alkalized to pH 9 by adding concentrated ammonium hydroxide solution and extracted 3 times with 10 ml of ethyl acetate each containing 10% of ether. The combined organic phase is washed with saturated sodium chloride solution, dried over anhydrous magnesium sulphate and evaporated. The thus-obtained oily product is rubbed with 4 ml of ether, the formed yellow substance is filtered by suction and purified by using preparative TLC (at this point the product weighs 0.14 g) on a KG—$PF_{254+366}$ sheet by means of a developing system containing dichloromethane and methanol in a volume ratio of 20:2. The $R_f$ value of 15',20'-anhydro-11'-nitrovincristine is lower than that of the starting 11'-nitrovincristine. The elution is carried out with a mixture of dichloromethane and methanol in a volume ratio of 20:4.

The aimed product is obtained in a yield of 60 mg (30%); m.p: 220°C (with decomposition) after recrystallization from methanol; $[\alpha]_{546}^{22} = +133.3°$ (c = 0.36, chloroform).

IR (KBr, $cm^{-1}$): 3400 (OH), 1740 (ester CO), 1670 (NCHO).

MS 851 ($M^+$, $C_{46}H_{53}N_5O_{11}$).

Method b)

A solution containing 0.06 ml (0.09 g, 1.44 mmoles) of fuming nitric acid in 0.28 ml of glacial acetic acid is dropped to a solution containing 0.40 g (0.49 mmole) of 15',20'-anhydrovincristine in a solvent mixture containing 0.93 ml of glacial acetic acid and 1.2 ml of abs. chloroform at −20°C under stirring. Thereupon, the stirring is continued at the same temperature for additional 3 hours, then the mixture is poured into 20 ml of ice-water, alkalized to pH 9 by adding concentrated ammonium hdyroxide solution and extracted 3 times with 6 ml of dichloromethane each. The organic phases are combined, dried over anhydrous magnesium sulphate, then filtered and evaporated under reduced pressure. The thus-obtained residue weighing 0.45 g is purified by using preparative TLC on a KG—$PF_{254+366}$ sheet by means of a developing system containing dichloromethane and methanol in a volume ratio of 20:2. The elution is carried out with a mixture of dichloromethane and methanol in a volume ratio of 20:4 to give a yield of 70 mg (16%) of the aimed product, the physical and chemical characteristics of which are in complete agreement with those of the product obtained by the above method a).

## Example 5

### Preparation of 11'-nitrovincristine sulphate

The pH value of a solution containing 0.60 g (0.69 mmole) of 11'-nitrovincristine base in 3 ml of ethanol is adjusted to 5 by adding an ethanolic solution containing 2% of concentrated sulphuric acid under cooling with ice. The thus-formed sulphate salt is precipitated by adding 25 ml of ether, filtered by suction and washed with ether to give 0.61 g (91%) of the aimed sulphate salt.

**Claims**

1. Nitro derivatives of vinblastine-type bis-indoles of the general formula

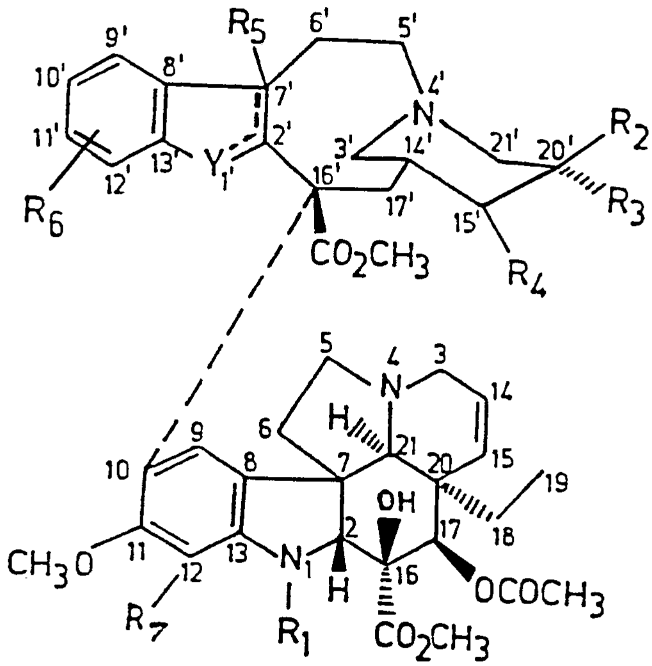

I,

wherein

$R_1$ stands for a methyl or a formyl group,

$R_2$ stands for a hydroxy or ethyl group of β-position,

$R_3$ means an ethyl group of α-position,

$R_4$ represents a hydrogen atom or

$R_2$ and $R_4$ together represent an oxygen bridge or a double bond,

$R_5$, $R_6$ and $R_7$ stand for a nitro group or a hydrogen atom with the proviso that simultaneously only at most two of them may stand for [a] hydrogen atom(s), and

Y stands for —N= when $R_5$ stands for a nitro group thus the dotted line between Y and the carbon atom 2′ representing a valence bond and the dotted line between the carbon atoms 2′ and 7′ having no meaning and

Y stands for

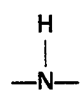

when $R_5$ stands for a hydrogen atom thus the dotted line between Y and the carbon atom 2′ having no meaning and the dotted line between the carbon atoms 2′ and 7′ representing a valence bond, as well as their acid addition salts.

2. 12-(Nitro)-vinblastine, 11′-(nitro)-vincristine, 9′-(nitro)-vincristine, 7′-(nitro)-vincristine, N-(formyl)-11′-(nitro)-leurosine, N-(formyl)-7′-(nitro)-leurosine and 15′,20′-(anhydro)-11′-(nitro)-vincristine as well as the acid addition salts of these compounds.

3. A process for preparing the compounds according to claim 1 or 2, characterized in that
a) a compound of the general formula

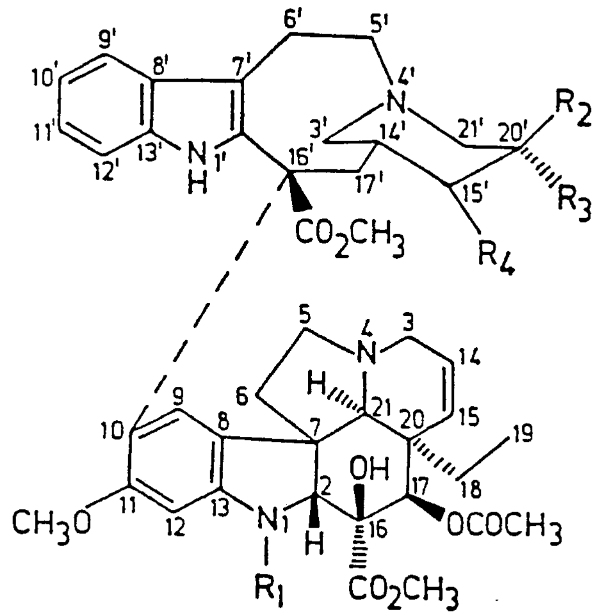

II,

wherein $R_1$, $R_2$, $R_3$ and $R_4$ are the same as defined in claim 1, is nitrated in an organic solvent, preferably in a chlorinated hydrocarbon, in the presence of an organic acid, preferably acetic acid, and optionally

(b) a compound of the general formula I, wherein $R_1$, $R_3$, $R_5$, $R_6$, $R_7$ and Y are the same as defined in claim 1, $R_2$ means a hydroxy group and $R_4$ represents a hydrogen atom, is dehydrated and the thus obtained compound is oxidized, whereby the meaning of $R_2$ and $R_4$ together is changed to an oxygen bridge

and, if desired, the thus obtained product is transformed to an acid addition salt thereof.

4. A process according to claim 3 for the preparation of 11'-(nitro)-vincristine, characterized in tha vincristine base as reacted with fuming nitric acid in the presence of chloroform and acetic acid and the formed product is separated.

5. Pharmaceutical compositions characterized by a content of 1 or more compound(s) according to claim 1 or 2 as [an] active principle(s), suitably in admixture with 1 or more usual pharmaceutical carrier(s) and/or additive(s).

**Patentansprüche**

1. Nitroderivate von Bisindolen vom Vinblastintypus, mit der allgemeinen Formel

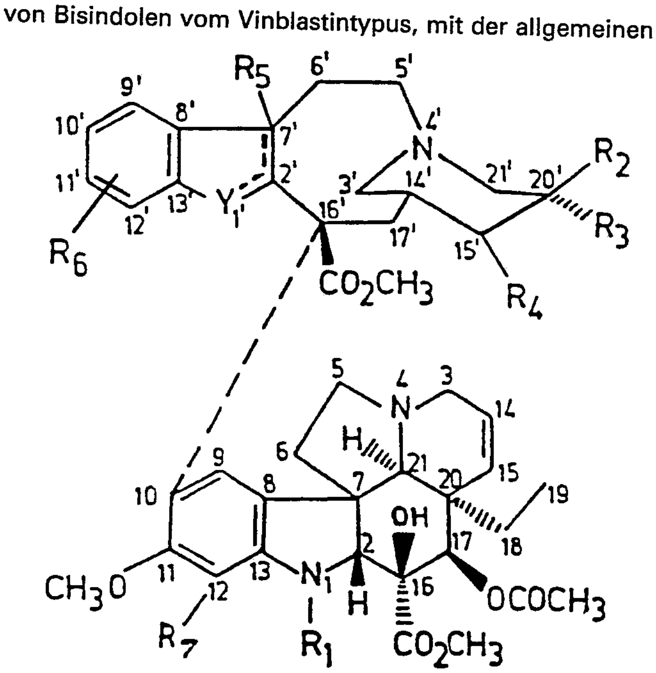

I,

in der

$R_1$ für eine Methyl- oder Formylgruppe steht,

$R_2$ für eine Hydroxy- oder Ethylgruppe in β-Stellung steht,

$R_3$ eine Ethylgruppe in α-Stellung bedeutet,

$R_4$ ein Wasserstoffatom darstellt oder

$R_2$ und $R_4$ zusammen eine Sauerstoffbrücke oder eine Doppelbindung darstellen,

$R_5$, $R_6$ und $R_7$ für eine Nitrogruppe oder ein Wasserstoffatom stehen, wobei gleichzeitig höchstens zwei von ihnen für (ein) Wasserstoffatom (e) stehen und

Y für —N= steht, wenn $R_5$ für eine Nitrogruppe steht, so daß dann die gestrichelte Linie zwischen Y und dem Kohlenstoffatom 2′ eine Valenzbindung darstellt und die gestrichelte Linie zwischen den Kohlenstoffatomen 2′ und 7′ keine Bedeutung hat, und

Y für

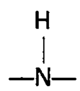

steht, wenn $R_5$ für ein Wasserstoffatom steht, so daß dann die gestrichelte Linie zwischen Y und dem Kohlenstoffatom 2′ keine Bedeutung hat und die gestrichelte Linie zwischen den Kohlenstoffatomen 2′ und 7′ eine Valenzbindung darstellt,

sowie deren Säureadditionssalze.

2. 12-(Nitro)-vinblastin, 11′-(Nitro), vincristin, 9′-(Nitro)-vincristin, 7′-(Nitro)-vincristin, N-(Formyl)-11′-(nitro)-leurosin, N-(Formyl)-7′-(nitro)-leurosin und 15′,20′-(Anhydro)-11′-(nitro)-vincristin sowie die Säureadditionssalze dieser Verbindungen.

3. Verfahren zum Erzeugen der Verbindungen nach Anspruch 1 oder 2, dadurch gekennzeichnet, daß

a) eine Verbindung der allgemeinen Formel

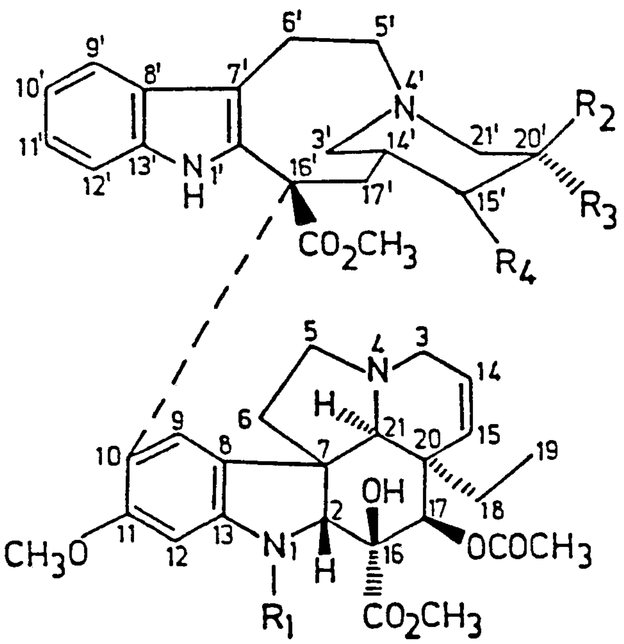

II,

in der $R_1$, $R_2$, $R_3$ und $R_4$ die im Anspruch 1 angegebenen Bedeutungen haben, in einem organischen Lösungsmittel, vorzugsweise in einem chlorierten Kohlenwasserstoff, in Gegenwart einer organischen Säure, vorzugsweise Essigsäure, nitriert wird, und gegebenenfalls

b) eine Verbindung der allgemeinen Formel I, in der $R_1$, $R_3$, $R_5$, $R_6$, $R_7$ und Y die im Anspruch 1 angegebenen Bedeutungen haben, $R_2$ eine Hydroxygruppe bedeutet und $R_4$ ein Wasserstoffatom darstellt, dehydriert wird und die so erhaltene Verbindung oxidiert wird, so daß die Bedeutung von $R_2$ und $R_4$ zusammen zu einer Sauerstoffbrücke geändert wird,

und gegebenenfalls das so erhaltene Produkt in ein Säureadditionssalz des Produkts umgewandelt wird.

4. Verfahren nach Anspruch 3 zum Erzeugen von 11′-(Nitro)-vincristin, dadurch gekennzeichnet, daß eine Vincristinbase in Gegenwart von Chloroform und Essigsäure mit rauchender Salpetersäure umgesetzt und das dabei gebildete Produkt abgetrennt wird.

5. Pharmazeutische Zusammensetzungen, gekennzeichnet durch einen Gehalt an 1 oder mehreren Verbindung(en) nach Anspruch 1 oder 2 als (einem) Wirkstoff(en), zweckmäßig im Gemisch mit 1 oder mehreren üblichen pharmazeutischen Träger(n) und/oder Zusatzstoff(en).

## Revendications

1. Dérivés nitrés de bis-indoles de type vinblastine de formule générale:

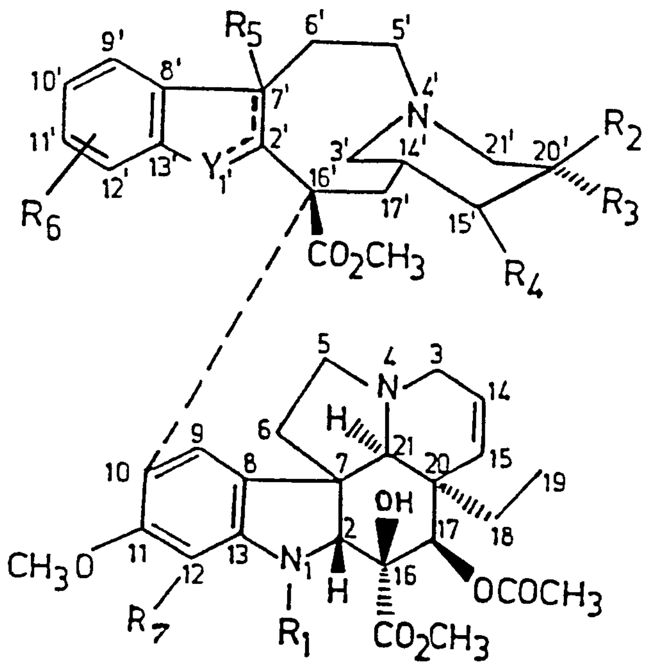

I,

dans laquelle:

$R_1$ représente un groupe méthyle ou formyle,

$R_2$ représente un groupe hydroxy ou éthyle en position β,

$R_3$ représente un groupe éthyle en position α,

$R_4$ représente un atome d'hydrogène, ou

$A_2$ et $R_4$ représentent ensemble un pont oxygène ou une double liaison,

$R_5$, $R_6$ et $R_7$ sont un groupe nitro ou un atome d'hydrogène, à condition qu'au maximum deux d'entre eux puissent être simultanément un atome d'hydrogène, et

Y représente —N= lorsque $R_5$ est un groupe nitro, la ligne en pointillés entre Y et l'atome de carbone 2' représentant alors une liaison de valence et la ligne en pointillés entre es atomes de carbone 2' et 7' n'ayant aucune signification et

Y représente

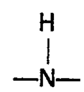

lorsque $R_5$ est un atome d'hydrogéne, la ligne en pointillés entre Y et l'atome de carbone 2' n'ayant alors aucune signification et la ligne en pointillés entre les atomes de carbone 2' et 7' représentant une liaison de valence,

ainsi que leurs sels d'addition acides.

2. Dérivées suivant la revendication 1, caractérisées en ce qu'il s'agit de a 12-nitro-vinblastine, la 11'-nitro-vincristine, la 9'-nitro-vincristine, la 7'-nitro-vincristine, la N-formyl-11'-nitroleurosine, la N-formyl-7'-nitro-leurosine et la 15',20'-anhydro-11'-nitro-vincristine, ainsi que de eurs sels d'addition acides.

3. Procédé pour préparer les composés suivant la revendication 1 ou 2, caractérisé en que:
a) un composé de formule générale

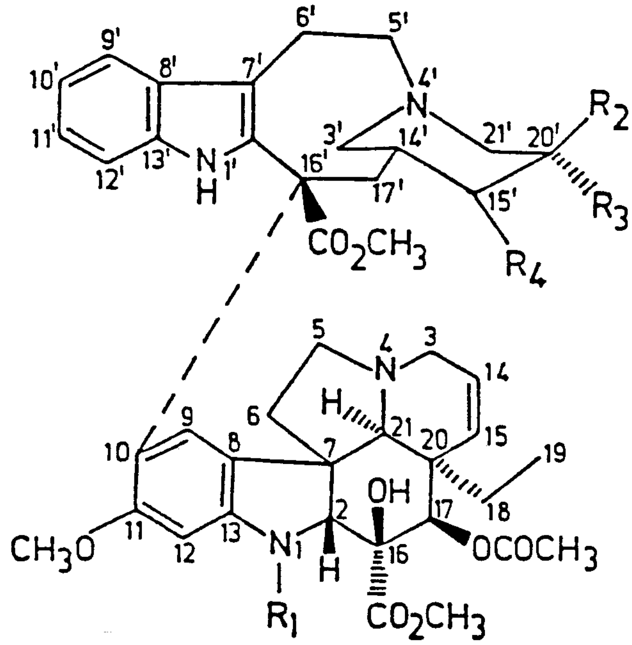

II,

dans laquelle $R_1$, $R_2$, $R_3$ et $R_4$ sont tels que définis dans la revendication 1, est nitré dans un solvant organique, de préference dans un hydrocarbure chloré, en présence d'un acide organique, de préférence l'acide acétique, et éventuellement

b) un composé de formule générale 1, dans laquelle $R_1$, $R_3$, $R_5$, $R_6$, $R_7$ et Y sont tels que définis dans la revendication 1, $R_2$ est un groupe hydroxy et $R_4$ est un atome d'hydrogène, est déshydraté et le composé ainsi obtenu est oxydé, de telle sorte que $R_2$ et $R_4$ représentent ensemble un point oxygène,
et, si cela est désiré, le produit ainsi obtenu est transformé en l'un de ses sels d'addition acides.

4. Procédé suivant la revendication 3 pour la préparation de la 11'-nitro-vincristine, caractérisé en ce que la vincristine base est traitée avec de l'acide nitrique fumant en présence de chloroforme et d'acide acétique, et que le produit formé est séparé.

5. Compositions pharmaceutiques, caractérisées en ce qu'elles contiennent un ou plusieurs composés suivant la revendication 1 ou 2 en tant que composant(s) actif(s), convenablement en mélange avec un ou plusieurs supports et/ou additifs pharmaceutiques.